# EUROPÄISCHE PATENTSCHRIFT

(11) **EP 1 506 957 B1**
(45) Veröffentlichungstag und Bekanntmachung des Hinweises auf die Patenterteilung: **24.08.2016**
(21) Anmeldenummer: 04016412.1
(22) Anmeldetag: 13.07.2004
(51) Int. Cl.: C07C 263/10, C07C 263/20, C07C 265/14, C08G 18/76

(54) **Herstellung von Mischungen von Di-und Polyisocyanaten der Diphenylmethanreihe mit hohen Gehalten an4,4'-Methylendiphenyldiisocyanat und 2,4'-Methylendiphenyldiisocyanat**
Process for the preparation of mixtures of di- and polyisocyanates of the diphenylmethane series with a high amount of 4,4'-methylenediphenyldiisocyanate and 2,4'-methylenediphenyldiisocyanate
Procédé pour la préparation de mélanges de di- et polyisocyanates de la série de diphenylmethane avec un taux élevé de 4,4'-methylenediphenydiisocyanate et 2,4'-methylenediphenyldiisocyanate

(30) Priorität: 25.07.2003 DE 10333929
(43) Veröffentlichungstag der Anmeldung: 16.02.2005
(73) Patentinhaber: Covestro Deutschland AG, 51373 Leverkusen (DE)
(72) Erfinder: Pirkl, Hans-Georg, Dr., 51377 Leverkusen (DE); Liman, Ulrich, Dr., 40764 Langenfeld (DE); Vieler, Robert, Dr., 41542 Dormagen (DE); Echterhoff, Ralf, Dr., 41539 Dormagen (DE)
(74) Vertreter: Levpat

(56) Entgegenhaltungen:
- EP-A- 1 270 544
- WO-A-99/40059
- WO-A1-02/070581
- WO-A1-2004/056757
- DE-A- 1 593 638

## Beschreibung

Die vorliegende Erfindung betrifft ein Verfahren zur Herstellung von Mischungen von Di- und Polyisocyanaten der Diphenylmethanreihe mit hohen Gehalten an 4,4'- und 2,4'-Methylendiphenyldiisocyanat sowie ihre Verwendung zur Herstellung von Polymeren.

Es ist bekannt, dass Di- und Polyisocyanate der Diphenylmethanreihe (MDI) durch Phosgenierung der entsprechenden Di- und Polyamine der Diphenylmethanreihe (MDA) hergestellt werden. Die Di- und Polyamine der Diphenylmethanreihe selbst werden durch Kondensation von Anilin und Formaldehyd hergestellt. Durch Phosgenierung der Diamine der Diphenylmethanreihe erhält man die entsprechenden Diisocyanate 2,2'-MDI, 2,4'-MDI und 4,4'-MDI, die in der Fachwelt als 2-Kern-MDI (Diisocyanate der Diphenylmethanreihe) beschrieben werden. Während der Kondensation von Anilin und Formaldehyd reagiert das 2-Kern-MDA (Methylendiphenyldiamin MDA) jedoch auch noch weiter mit Formaldehyd und Anilin zu höherkernigen MDA-Typen, die nach der Phosgenierung den Mehrkerngehalt im polymeren MDI (Polyisocyanate der Diphenylmethanreihe) darstellen. Für viele praktische. Produktanwendungen ist es nun bevorzugt, einen hohen Anteil an 2-Kern MDI zu erhalten. Dies kann nach dem derzeitigen Stand der Technik auf zwei verschiedenen Wegen erzielt werden.
1. Man setzt Anilin bei der sauerkatalysierten Kondensation von Anilin und Formaldehyd in großem Überschuss ein, trennt das überschüssige Anilin aus dem Reaktionsgemisch ab und führt das überschüssige Anilin zurück. Durch den hohen Überschuss an Anilin während der Kondensation erhält man ein MDA-Gemisch mit hohem Anteil an 2-Kern MDA. Je höher das bei der Reaktion eingesetzte Anilin/Formaldahyd-Verhältnis eingestellt wird, desto höher ist der im Reaktionsprodukt enthaltene 2-Kern-Gehalt. Weiterhin kann das Verhältnis von 2,4'-MDA zu 4,4'-MDA durch die Konzentration des Säurekatalysators beeinflußt werden. Hohe Katalysatorkonzentrationen begünstigen 4,4'-MDA, niedrige 2,4'-MDA (EP-158059-B1 und EP-3303-B1). Solche MDA-Typen werden im Weiteren Polymer-A- oder Polymer-B-Typen genannt. Durch Phosgenierung der vorher gezielt hergestellten MDA-Typen kann man dann die gewünschte MDI-Zusammensetzung erhalten.
2. Man stellt polymeres MDA konventionell säurekatalysiert aus Anilin und Formaldehyd her. Das polymere MDA wird phosgeniert und destillativ in eine stark Monomer-reiche und eine Polymer-reiche Fraktion zerlegt. Die polymere MDI-Fraktion steht als polymeres MDI-Verkaufsprodukt zur Verfügung. Die monomere MDI-Fraktion wird nach dem Stand der Technik destillativ oder per Kristallisation in die Isomere 4,4'-MDI und eine Mischung von etwa 50% 2,4'-MDI und 50% 4,4'-MDI zerlegt. Die beiden monomeren Produkte werden verkauft oder zu Mischprodukten mit polymer-MDI mit hohen Gehalten an 2-Kern-MDI und im richtigen Isomerenverhältnis weiterverarbeitet.

In beiden Fällen ist der Aufwand für die Herstellung der MDI-Gemische mit hohem Anteil an 2-Kern MDI durch Investition und Energieverbrauch erheblich.

Im ersten Fall müssen bei der Kondensation von Anilin und Formaldehyd hohe Überschüsse an Anilin im Kreislauf geführt, durch Destillation vom Reaktionsgemisch abgetrennt und anschließend zurückgeführt werden.

Im zweiten Fall entstehen bei der Kondensation von Anilin und Formaldehyd mit geringeren Überschüssen an Anilin neben den gewünschten Diaminen beträchtliche Mengen an höherkernigen MDA-Typen, die anschließend zusammen mit dem Diaminen phosgeniert werden. Um aus dem Gemisch von Di- und Polyisocyanaten (2-Kern MDI und höherkernige MDI-Typen) die Diisocyanate zu erhalten, müssen die Diisocyanate aus dem Gemisch von Di- und Polyisocyanaten abdestilliert werden (Monomer/Polymertrennung). Nach dieser Monomerabtrennung müssen die Isomere destillativ und/oder durch Kristallisation durch hohen apparativen und energetischen Aufwand voneinander getrennt werden. Anschließend werden die mühsam getrennten Isomere in hochmonomerhaltigen Fertigprodukten wieder miteinander abgemischt.

Das dabei nach dem Verfahren nach dem Stand der Technik bei der Monomer/Polymertrennung erhaltene rohe Monomer-Destillat (Monomer Roh, im Wesentlichen bestehend aus Diisocyanat) enthält jedoch noch unerwünscht hohe Konzentrationen an Nebenkomponenten. Beispielsweise sind in diesem rohen MDI-Monomer-Gemisch ein Vielfaches der von den Polyurethan-Herstellern häufig geforderten maximalen Konzentration von maximal 50 ppm Monochlorbenzol und maximal 20 ppm Phenylisocyanant, bevorzugt maximal 20 ppm Monochlorbenzol und maximal 10 ppm Phenylisocyanant enthalten. Weiterhin enthält konventionelles rohes MDI-Monomergemisch eine erhebliche Menge an Uretdion (dimeres para-MDI), das in größeren Konzentrationen zur Produkteintrübung und Feststoffausfall im MDI führt. Daher müssen die in der Monomer/Polymertrennung abdestillierten Diisocyanate in nachfolgenden Destillationsstufen aufwendig von den Nebenkomponenten gereinigt werden.

Im Markt werden spezielle MDI-Typen mit spezifizierten Gehalten an Isomeren, also 2,4'-MDI, 2,2'- MDI und 4,4'-MDI gehandelt. Daher werden nach dem Stand der Technik die entsprechenden Zusammensetzungen der im Handel erhältlichen MDI-Typen mit den niedrigen Gehalten an Nebenkomponenten durch aufwendige, mehrstufige destillative und/oder kristallationstechnische Isomerentrennung hergestellt. Die für eine spezielle Anwendung benötigte Isocyanatzusammensetzung wird dann durch Abmischen dieser MDI-Typen erzeugt.

Für viele Anwendungen der MDI-Typen wird aber in der Herstellung von Polymeren .keine sehr hohe Reinheit an 4,4'-MDI bei den abzumischenden Rohstoffen, also beispielsweise kein sehr niedriger Gehalt an 2,2'-MDI, benötigt. Die Verwendung eines durch mehrere aufwendige Destillationsstufen hergestellten reinen 4,4'-MDI-Typs mit sehr geringen Gehalten an 2,2'-MDI zum Abmischen mit anderen MDI-Typen zur Herstellung einer für die spezielle Anwendung geeigneten Isocyanatzusammensetzung ist daher aus energetischer Sicht unbefriedigend.

Aus dem Stand der Technik ist weiterhin folgendes bekannt:
Die direkte Herstellung von MDI-Mischprodukten durch eine gezielte MDA-Synthese beschreiben beispielsweise die folgenden beiden Arbeiten. Keggenhoff, Maehlmann & Eifler (EP-158059 B1) stellten ein hoch 4,4'-MDA-haltige MDA-Mischung mit ca. 80 % 4,4'-MDA und ca. 10 % 2,4'-MDA bei einem 2-Kern-Gehalt von ca. 90 % her. Dagegen konnten Eifler & Ellendt (EP-3303 B1) ein hoch monomerhaltiges MDA mit 88 % 2-Kern mit 19 % 2,2'-MDA, 36 % 2,4'-MDA und 45 % 4,4'-MDA herstellen. Zur Herstellung dieser Produkte sind hohe molare Verhältnisse von Anilin/Formaldehyd > 8 notwendig, so dass große Anilinmengen zurückgeführt werden müssen. Weiterhin sind für viele dieser Typen hohe Verbräuche an HCl (zur Katalyse) und NaOH (zur Neutralisation des Katalysators) erforderlich. Vor allem Produkte mit hohem 4,4'-MDA-Gehalt weisen einen sehr hohen spezifischen HCl-Katalysatorverbrauch auf. Insbesondere die Herstellung hoch 2,4'-MDI-haltiger, monomerreicher MDA-Typen führt zu einem hohen, vielfach unerwünschten Nebenausbeute an 2,2'-MDI. Wegen seiner Reaktionsträgheit ist 2,2'-MDI in vielen Anwendungen in hohen Konzentrationen nicht erwünscht.

Die Herstellung von polymeren und monomeren MDI-Produkten ist in der Literatur allgemein bekannt. Die Herstellung des polymeren MDA mit 2-Kern-Gehalten von 46 bis 65 % kann beispielsweise nach DE-2750975-A1 oder DE-2517301 A1 erfolgen. In der Literatur werden ausführlich die beiden technisch hauptsächlich genutzen Verfahren der Destillation (DE-3145010 A1) und der Kristallisation (EP-A-482490) zur Isomerentrennung der MDI-Monomere beschrieben. In keinem der Arbeiten wird jedoch der direkte Einsatz der rohen monomeren MDI-Fraktion aus der Monomer/ Polymertrennung als Rohstoffquelle für MDI-Abmischungen beschrieben. Die Fachwelt konzentriert sich dagegen auf die möglichst ökonomische Herstellung der feingereinigten monomeren Isomerenmischungen mit > 98 % 4,4'-MDI und einer ca. 50 % 2,4'- und ca. 50 %igen 4,4'-MDI-Mischung, die gegebenenfalls bis zu 2,5 % 2,2'-MDI enthalten kann (M. Stepanski, P. Faessler: "New hybrid process for purification and separation of MDI isomers",Sulzer Chemtech, Presentation at the Polyurethanes Conference 2002 in Salt Lake City, 10/2002).

Die Herstellung von hoch monomerhaltigen MDI-Gemischen über die Abmischung von polymeren MDI-Produkten und mehrfach destillierten, monomeren Produkten ist ein in der Polyurethan-Industrie gängiges Verfahren. Beispielsweise wird dies zur Herstellung niederviskoser MDI-Abmischprodukte in US-A-5,258,417 eingesetzt.

Die Reinigung von rohen, polymeren MDI-Gemischen ist prinzipiell in vielen Arbeiten untersucht worden. Beispielsweise wird mittels chemischen Zusätzen versucht, Verunreinigungen zu beseitigen (US-A-3,925,437, US-A-3,793,362, DE-A-2316028). In DD-A-118,105 und GB-A-1,114,690 wird mittels Lösungsmittelzusatz versucht, chemisch gebundene Verunreinigungen zu entfernen. In DD-A-271,820 wird eine Strippung von MDI und TDI (Toluylendiisocyanat) vorgeschlagen, die jedoch eine deutliche Zersetzung der Einsatzstoffe mit 0,1 bis 10 Gew.-% bewirkt und wesentlich das Ziel der Entfernung sehr hartnäckiger, chemisch gebundener Verunreinigungen verfolgt.

In US-A-3,857,871 wird ein Stripp-Verfahren für polymeres MDI offenbart, welches zu einer Reduzierung von Acidität und hydrolysierbarem Chlor im Produkt führt. In GB-A-1,362,708 wird ein Verfahren zur Reinigung von polymerem MDI beschreiben, welches zwar die Menge an hydrolysierbarem Chlor signifikant erniedrigt. Das Produkt enthält jedoch immer noch 0,1% Lösungsmittel.

WO-A-99/40059 beschreibt ein Verfahren zur Herstellung von Methylendi(phenylamin) durch Umsetzung von Anilin mit Formaldehyd in Gegenwart saurer Katalysatoren, die mit diesem Verfahren herstellbaren Gemische enthaltend Methylendi(phenylamin), ein Verfahren zur Herstellung von Polyisocyanaten durch Phosgenierung dieser Gemische enthaltend Methylendi(phenylamin) und derart erhältliche Polyisocyanate.

WO-A-02/070581 beschreibt ein Verfahren zur Herstellung von MDI, umfassend die Schritte A) Umsetzung von Anilin und Formaldehyd in Gegenwart einer Säure als Katalysator zu Methylen (diphenyldiaminen) und Polymethylen-polyphenylen-polyaminen, wobei das molare Verhältnis von Säure zu Amin 0,2 oder kleiner ist, B) Umsetzung des in Schritt A) erhaltenen Gemisches mit Phosgen zu Methylen (diphenyldiisocyanaten) und Polymethylen-polyphenylenpolyisocyanaten, C) Auftrennen des Gemisches aus Schritt B) in Monomer-MDI und PMDI und D) Auftrennen des in Schritt C) erhaltenen Monomer-MDI zur Gewinnung von 2,4'-MDI.

DE1953638 beschreibt ein Verfahren zur gleichzeitigen Herstellung von Methylen-bis(phenylisocyanat) und Polymethylenpolyphenyl-isocyanat-Mischungen mit variierenden Anteilen an Methylen-bis-phenylisocyanat.

Die Aufgabe der vorliegenden Erfindung ist daher, ein einfaches und energiesparendes Verfahren zur Herstellung von hoch Monomer-haltigen MDI-Gemischen und ein Verfahren zur Herstellung von MDI-Gemischen mit einem gewünschten Verhältnis der MDI-Isomere zur Verfügung zu stellen, das einen sehr niedrigen Anteil an Nebenkomponenten, insbesondere in Bezug auf das Lösungsmittel, Phenylisocyanat, Uretdion und Phosgen enthält.

Die Erfindung betrifft ein Verfahren zur Herstellung einer Fraktion von Diisocyanaten der Diphenylmethanreihe enthaltend mindestens 95 Gew.-% 2-Kern-Methylendiphenyldiisocyanat, bei dem man
a) Anilin und Formaldehyd in Gegenwart eines sauren Katalysators zu Di- und Polyaminen der Diphenylmethanreihe enthaltend 2-Kern-Methylendiphenyldiamin umsetzt, und
b) die Di- und Polyamine der Diphenylmethanreihe enthaltend 2-Kern-Methylendiphenyldiamin gegebenenfalls in Gegenwart eines Lösungsmittels phosgeniert, wobei man ein rohes Di- und Polyisocyanat erhält, und
c) aus dem rohen Di- und Polyisocyanat eine Fraktion enthaltend mindestens 95 Gew.-% 2-Kern-Methylendiphenyldiisocyanat mit einem Gehalt an 4,4'-MDI- größer 60 Gew.-%, einem Gehalt an 2,4'-MDI von 4 bis 35 Gew.-% und einem Gehalt an 2,2'-MDI von 0,01 bis 10 Gew.-%, bezogen auf die Masse der Fraktion, und maximal 20 ppm Phenylisocyanat sowie gegebenenfalls maximal 50 ppm Lösungsmittel abtrennt, wobei die Abtrennung der
   Fraktion in einem einzigen Destillationsschritt in einer Destillationskolonne mit Seitensstromentnahme, bei der die Fraktion im Seitenstrom der Kolonne und Leichtsieder am Kopf der Kolonne abgetrennt werden, erfolgt.

Bevorzugt trennt man in Schritt c) aus dem rohen Di- und Polyisocyanat eine Fraktion enthaltend mindestens 99 Gew.-% 2-Kern-Methylendiphenyldiisocyanat mit einem Gehalt an 4,4'-MDI größer 76 Gew.-%, einem Gehalt an 2,4'-MDI von 5 bis 22 Gew.-% und einem Gehalt an 2,2'-MDI von 0,2 bis 3 Gew.-%, bezogen auf die Masse der Fraktion, und maximal 10 ppm Phenylisocyanat sowie gegebenenfalls maximal 20 ppm Lösungsmittel ab.

Die angegebenen maximalen Konzentrationen an Phenylisocyanat und Lösungsmittel beziehen sich dabei ebenfalls auf die Masse der gesamten Fraktion.

Dabei bedeutet die Abtrennung der Fraktion in einem einzigen Destillationsschritt, dass das abgetrennte MDI nur in diesem einzigen Destillationsschritt komplett verdampft und an einer anderen Stelle der Kolonne wieder kondensiert und als Fraktion gewonnen wird.

Insbesondere bedeutet die Abtrennung der Fraktion in einem einzigen Destillationsschritt daher auch, dass gemäß dem erfindungsgemäßen Verfahren nach der Abtrennung der Fraktion in dem einen einzigen Destillationsschritt aus dem rohen Di- und Polyisocyanat im Wesentlichen keine weitere Trennung der isomeren MDI-Diisocyanate mehr erfolgt.

Nach dem erfindungsgemäßen Verfahren gelingt es, hoch Monomer-haltige MDI-Gemische mit einem hohen Gehalt an 2-Kern-MDI von mindestens 95 Gew.-% bezogen auf die Masse der Fraktion mit geringen Anteilen an Nebenkomponenten mit einem deutlich geringeren Aufwand an Energie zur Verfügung zu stellen. Der Energiebedarf ist dabei gegenüber den Verfahren nach dem Stand der Technik reduziert, da auf die Kondensation von Anilin und Formaldehyd mit großen Überschüssen an Anilin und dessen nachfolgender Destillation und Rückführung verzichtet wird. Gleichzeitig wird nach dem erfindungsgemäßen Verfahren jedoch auch bereits bei der Destillation des aus der Phosgenierung kommenden Gemisches aus Di- und Polyisocyanaten die Menge an unerwünschten Nebenkomponenten in nur einem Destillationsschritt reduziert, so dass nachfolgende Destillationsschritte unnötig werden. Auf die Isomerentrennung durch mehrstufige Destillation, bei der nach dem Stand der Technik gleichzeitig die unerwünschten Nebenkomponenten entfernt werden, wird nach dem erfindungsgemäßen Verfahren verzichtet.

Bei der Isomerendestillation von MDI nach dem Stand der Technik wird ein hoher technischer Aufwand betrieben, um auch Spurenkomponenten, beispielsweise hydrolysierbare Chlorkomponenten, zu zerstören. Solche Spurenkomponenten stören aber in hoch monomerhaltigen MDI-Gemischen nicht. Um die noch enthaltenen Spurenkomponenten zu entfernen, wird typischerweise monomeres MDI während der Isomerendestillation ca. 4 bis 6mal verdampft und kondensiert, bevor es als reines 4,4'-MDI verkauft werden kann. Hingegen ist der Energieaufwand zur erfindungsgemäßen Herstellung eines lösungsmittelarmen rohen monomerem MDI deutlich kleiner, da das MDI in einem einzigen Destillationsschritt hergestellt wird.

Die Herstellung der hoch Monomer-haltigen MDI-Fraktion mit einem hohen Gehalt an 2-Kem-MDI von mindestens 95 Gew.-% bezogen auf die Masse der Fraktion nach dem erfindungsgemäßen Verfahren gelingt durch ein Entfernen der störenden leichtsiedenden Nebenkomponenten wie Phenylisocyanat sowie gegebenenfalls Lösungsmittel bereits in der Monomer/Polymertrennung. Die Monomer/Polymertrennung in Schritt c) wird dabei bevorzugt bei absoluten Drücken von 1 bis 50 mbar am Kopf der Destillationskolonne und bei Temperaturen von 100 bis 300°C durchgeführt.

Bei Verzicht auf die Leichtsiederabtrennung durch Strippung ist die Monomer/Polymerabtrennung als Destillationskolonne mit einer Seitenstromentnahme ausgeführt. Hierbei kann das saubere MDI-Monomergemisch im Seitenstrom der Kolonne und die Leichtsieder am Kopf der Kolonne abgetrennt werden. Die Leichtsieder können dabei zurückgeführt oder bevorzugt in einer separaten Leichtsiederabtrennkolonne von Lösungsmittel und Phenylisocyanat befreit werden.

Die Entfernung von Leichtsiedern kann aber auch so verbessert werden, dass der Monomer-/Polymerabtrennung praktisch kein Phenylisocyanat oder Lösungsmittel mehr zugeführt wird. Dies geschieht beispielsweise durch die Rückführung der Destillate der gegebenenfalls mehrstufigen Lösungsmittelentfemung vor der Monomer/Polymerabtrennung in die erste Stufe der Lösungsmittelabtrennung.

Ein geeignetes Verfahren zur Reinigung des rohen MDI-Monomergemisches kann auch eine Kristallisation sein.

Bei dem Verfahren ist das bei der Monomer/Polymertrennung aus der Destillationskolonne erhaltene Produkt bevorzugt mit einer schnellen Quench-Kühlung auf eine Temperatur von 35 bis 80°C abzukühlen, um den Gehalt an Uretdion gering zu halten. Hierzu existieren bereits geeignete Verfahren zur Quenchkühlung des kontinuierlichen MDI-Monomerstromes im Stand der Technik. Beispielsweise ist ein Kreislaufsystem mit Umwälzpumpe und Wärmeaustauscher bei einer Temperatur von z.B. 60°C geeignet, um das heiße Produkt von ca. 140 bis 200°C in kürzester Zeit auf 60°C abzukühlen und so die Uretdion-Bildung gering zu halten.

Die Erfindung betrifft weiterhin ein Verfahren zur Herstellung von Mischungen enthaltend Diisocyanate der Diphenylmethanreihe, bei dem man
a) eine Fraktion enthaltend mindestens 95 Gew.-% 2-Kern-Methylendiphenyldiisocyanat mit einem Gehalt an 4,4'-MDI größer 60 Gew.-%, einem Gehalt an 2,4'-MDI von 4 bis 35 Gew.-% und einem Gehalt an 2,2'-MDI von 0,01 bis 10 Gew.-%, bezogen auf die Masse der Fraktion, und maximal 20 ppm Phenylisocyanat sowie gegebenenfalls maximal 50 ppm Lösungsmittel nach dem Verfahren gemäß Anspruch 1 herstellt, und
b) die in Schritt a) hergestellte Fraktion mit einem oder mehreren Gemischen enthaltend Di- und / oder Polyisocyanate der Diphenylmethanreihe abmischt.

Die nach dem erfindungsgemäßen Verfahren hergestellte Fraktion von Diisocyanaten der Diphenylmethanreihe enthaltend mindestens 95 Gew.-% 2-Kern-MDI kann zur Abmischung mit höherkernigem (polymerem) MDI und den kommerziell verfüg-baren monomeren MDI Typen zu jeder beliebigen Mischung von MDI mit beliebig einstellbaren Anteilen an 2-Kern MDI und höherkernigem MDI und beliebig einstellbaren Anteilen der verschiedenen MDI Isomere eingesetzt werden.

Die nach dem erfindungsgemäßen Verfahren hergestellten Fraktionen von Diisocyanaten der Diphenylmethanreihe haben eine bevorzugte Zusammensetzung von:
- Summe 2-Kerngehalt: ≥ 95 Gew.-%
- 4,4'-MDI-Gehalt: > 60 Gew.%, bezogen auf die gesamte Zusammensetzung,
- 2,4'-MDI-Gehalt: 4 Gew.-% bis 35 Gew.-%, bezogen auf die gesamte Zusammensetzung,
- 2,2'-MDI-Gehalt: 0,01 Gew.-% bis 10 Gew.-%, bezogen auf die gesamte Zusammensetzung,
- Lösungsmittel-Gehalt: 0 - 50 ppm, bezogen auf die gesamte Zusammensetzung,
- Phenylisocyanat-Gehalt: 0 - 20 ppm, bezogen auf die gesamte Zusammensetzung,
- Uretdion-Gehalt: 0 - 0,5 Gew.-%, bezogen auf die gesamte Zusammensetzung Besonders bevorzugte Zusammensetzungen sind
- Summe 2-Kerngehalt: ≥ 99 Gew.-%
- 4,4'-MDI-Gehalt: > 76 Gew.-%, bezogen auf die gesamte Zusammensetzung,
- 2,4'-MDI-Gehalt: 5 Gew.-% bis 22 Gew.-%, bezogen auf die gesamte Zusammensetzung,
- 2,2'-MDI-Gehalt: 0,2 Gew.-% bis 3 Gew.-%, bezogen auf die gesamte Zusammensetzung,
- Lösungsmittel-Gehalt: 0 - 20 ppm, bezogen auf die gesamte Zusammensetzung,
- Phenylisocyanat-Gehalt: 0 - 10 ppm, bezogen auf die gesamte Zusammensetzung,
- Uretdion-Gehalt: 0 - 0,1 Gew.-%, bezogen auf die gesamte Zusammensetzung

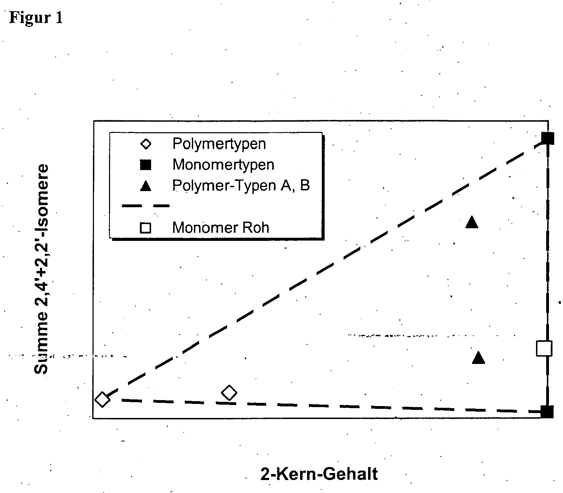

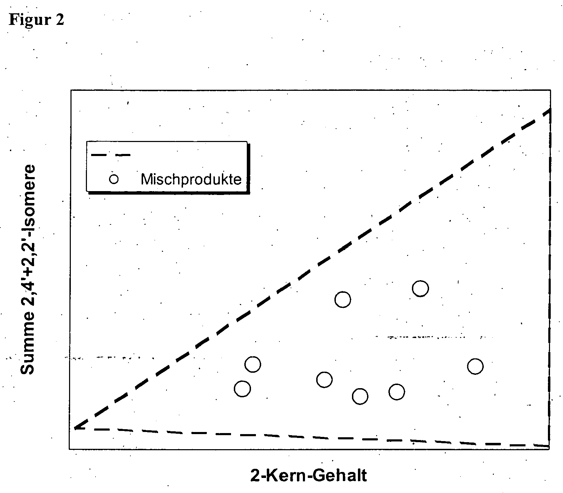

Die nach dem erfindungsgemäßen Verfahren hergestellten Fraktionen von Diisocyanaten der Diphenylmethanreihe enthaltend mindestens 95 Gew.-% 2-Kern-MDI können mit Polyisocyanaten bzw. anderen organischen Isocyanaten abgemischt werden. Die Figur 1 zeigt schematisch einige kommerziell verfügbare Ausgangsprodukte für die Herstellung von MDI- Abmischungen. Figur 2 zeigt einige exemplarische Abmischprodukte der MDI-Reihe im gleichen Parameterraum. Die horizontale Achse beschreibt dabei die Summe des 2-Kern-Gehaltes, die vertikale Achse die Summe der ortho-2-Kem-Gehalte (2,2'-MDI + 2,4'-MDI). Die gestrichelte Linie spannt dabei das Dreieck auf, innerhalb dessen Abmischungen aus den gegebenen Ausgangsprodukten hergestellt werden können.

Die nach dem erfindungsgemäßen Verfahren hergestellten Fraktionen von Diisocyanaten der Diphenylmethanreihe enthaltend mindestens 95 Gew.-% 2-Kern-MDI können mit einem oder mehreren Gemischen enthaltend aromatische Isocyanate abgemischt werden. Für die Abmischung können neben den bereits erwähnten, bevorzugt eingesetzten Gemischen enthaltend Di- und/oder Polyisocyanaten der Diphenylmethanreihe bevorzugt auch Gemische enthaltend Toluylendiisocyanat (zum Beispiel 2,4-TDI, 2,6-TDI) oder Gemische enthaltend Naphthalindiisocyanat (zum Beispiel 1,5-NDI) oder Gemische aus diesen Isocyanaten eingesetzt werden. Grundsätzlich können für die Abmischung aber auch andere aromatische und/oder aliphatische Mono-, Di-, Tri- oder höherfunktionellen Isocyanate eingesetzt werden.

Die nach dem erfindungsgemäßen Verfahren hergestellten Fraktionen von Diisocyanaten der Diphenylmethanreihe enthaltend mindestens 95 Gew.-% 2-Kern-MDI und die daraus hergestellten Abmischprödukte können zu den üblichen Modifizierungsreaktionen von Isocyanaten benutzt werden, wie z.B: zur Carbodiimidisierung und daraus resultierende Uretonimine, oder zur Herstellung von Prepolymeren aus OH-funktionellen Polyestern, C2/C3/C4-Polyethern, Uretdionen, Allophanaten, Biureten oder Harnstoffderivaten.

Die nach dem erfindungsgemäßen Verfahren hergestellten Fraktionen von Diisocyanaten der Diphenylmethanreihe enthaltend mindestens 95 Gew.-% 2-Kern-MDI finden Verwendung unter anderem als Abmischkomponente für andere Di- und Polyisocyanate der MDI-Reihe und/oder anderen Isocyanaten zur Herstellung von Isocyanatkomponenten für den Einsatz in der Herstellung von Polymeren. Die aus diesen Abmischprodukten herzustellende Fertigteile decken das gesamte Feld der Polyurethan-Chemie ab. Beispielhaft genannt sind hier:
- Hartschaumstoffe für die Isolier und Kühlgeräteindustrie
- Verpackungsschaum
- Weichschaum und Weichformschaum für Möbel und Autoindustrie
- Anwendungen im Spektrum der CASE-Anwendungen (Coatings, Adhesives, Sealants, Elastomers)
- Halbharte PU-Schaumstoffe

Die erfindungsgemäß hergestellten MDI-Abmischprodukte können durch einen deutlich verminderten apparativen und energetischen Aufwand bei der Destillation auf der MDI-Isomerentrennstufe beziehungsweise bei der Anilindestillation auf der MDA-Stufe hergestellt werden. Gleichzeitig kann der durchschnittliche Rohstoffverbrauch an HCl und NaOH auf der MDA-Stufe signifikant vermindert werden, wenn die Verwendung von Polymer-A- oder Polymer-B-Typen, die nach dem Stand der Technik mit großen spezifischen Mengen an HCl (zur Steuerung der Isomerenverteilung des MDA) und NaOH hergestellt werden, vermieden werden kann.

Bei vielen Abmischprodukten kann weiterhin der Gehalt an 2,2'-MDI reduziert werden, wenn hoch 2,2'-MDI-haltige Polymer-B-Typen nicht mehr direkt hergestellt und als Abmischkomponenten für hoch monomerhaltige Endprodukte eingesetzt werden. Durch Einsatz von 2,2'-MDI-armen MDI-Typen bei der Abmischung, wie der nach Anspruch 1 hergestellten Fraktion mit nur geringen Gehalten an 2,2'-MDI, können MDI-Produkte mit deutlich geringeren Gehalten an 2,2'-MDI- und somit verbesserter Reaktivität durch Abmischen hergestellt werden. Die daraus hergestellten Polymere zeigen daher auch verbesserte Polymereigenschaften.

### Beispiele:

Die Lösung der geschilderten erfinderischen Aufgabe wird anhand der folgenden Beispielen erläutert. Dabei sind alle %-Angaben auf Gewicht bezogen (Gew.-%).

Für die Herstellung von verkaufsfähigen MDI-Gemischen stehen die folgenden MDI-Rohstoffe mit der angegebenen Zusammensetzung zur Verfügung (MCB = Lösungsmittel Monochlorbenzol, PHI = Phenylisocyanat):

| Typ | 2,2'-MDI, | 2,4'-MDI | 4,4'-MDI, | Summe 2-Kern | MCB | PHI |
|---|---|---|---|---|---|---|
| Polymer-Typ (200mPas) | <0,2% | 3% | 39% | 42% | < 10 ppm | < 8 ppm |
| 4,4'-reiner-Typ | 0% | 1,5% | 98,5% | 100% | < 10 ppm | < 8 ppm |
| 2,4'-reicher Typ | < 2% | 55% | 44% | 100% | < 10 ppm | < 8 ppm |
| 2,2'-reicherTyp | 50% | 50% | 0% | 100% | < 10 ppm | < 8 ppm |
| Polymer-A-Typ | 0,7% | 11% | 78% | 90% | < 10 ppm | < 8 ppm |
| Polymer-B-Typ | 6% | 31% | 51% | 88% | < 10 ppm | < 8 ppm |
| Monomer Roh | 0,7% | 11% | 88% | > 99% | 250 ppm | 44 ppm |
| Monomer Roh rein* | 0,7% | 11% | 88% | > 99,5% | 15 ppm | 8 ppm |

| | | | | | | |
|---|---|---|---|---|---|---|
| * nach dem erfindungsgemäßen Verfahren hergestellte Fraktion | | | | | | |

Die beiden Polymer-Typen A und B werden mit hohen Anilin-Überschüssen hergestellt und erfordern damit eine aufwendige und kostenintensive Anilin-Destillation und -Rückführung. Dagegen müssen die MDI-Isomerendestillionsprodukte 4,4'-reiner, 2,4'-reicher und 2,2'-reicher Typ durch sehr aufwendige und mehrstufige Destillation aus dem aus der Phosgenierung stammenden Gemisch aus Di- und Polyisocyanaten hergestellt werden, um die unerwünschten Nebenkomponenten abzutrennen und um die geforderten Spezifikationen für die einzelnen 2-Kern MDI-Isomere (2,4'-MDI, 2,2'-MDI und 4,4'-MDI) einzustellen.

Die Typen Polymer-Typ sowie die Monomer Roh-Typen (Monomer Roh und Monomer Roh rein) sind dagegen mit geringem Aufwand und geringem Energieverbrauch aus dem aus der Phosgenierung erhaltenen Gemisch an Di- und Polyisocyanaten durch eine einzige Destillation zu erhalten. Der Polymer-Typ fällt dabei als Sumpfprodukt an und enthält große Anteile an höherkernigem MDI. Die Monomer Roh-Typen werden am Kopf oder als Seitenabzug abgezogen. Monomer Roh und Monomer Roh rein unterscheiden sich dabei dadurch, dass Monomer Roh rein nach dem erfindungsgemäßen Verfahren hergestellt wurde und daher nur noch sehr geringe Anteile an Nebenkomponenten wie Phenylisocyanat und gegebenenfalls Lösungsmittel aufweist und daher nicht mehr destillativ weiter aufgearbeitet werden muss.

### Beispiel 1 (nicht erfindungsgemäß):

Herstellung eines MDI-Mischproduktes aus 17 Gew.-% Polymer-Typ, 51 Gew.-% Polymer-Typ A und 32 Gew.-% Polymer-Typ B ergibt die folgende Produktqualität (MCB = Monochlorbenzol als Lösungsmittel)

| Typ | 2,2'-MDI | 2,4'-MDI | 4,4'-MDI | Summe 2-Kern-MDI | MCB |
|---|---|---|---|---|---|
| Mischung 1 | 3,9% | 16,3% | 62,5% | 82,7% | < 10 ppm |

Für die Herstellung von Mischung 1 wird zwar kein feindestilliertes Produkt (4,4'-reiner Typ, 2,2'-reicher Typ, 2,4'-reicher Typ) eingesetzt, dafür müssen aber 83 Gew.-% mit großem Aufwand hergestellte Polymer-Typen A und/oder B eingesetzt werden. Die Herstellung von Mischung 1 nach Beispiel 1 ist daher energetisch sehr aufwendig.

### Beispiel 2 (nicht erfindungsgemäß):

Herstellung eines MDI-Mischproduktes aus 36,4 % Polymer-Typ, 37,1 % 4,4-Typ und 26,5 % 2,4-Typ ergibt die folgende Produktqualität:

| Typ | 2,2'-MDI | 2,4'-MDI | 4,4'-MDI | Summe 2-Kern MDI | MCB |
|---|---|---|---|---|---|
| Mischung 2 | 0,3% | 16,3% | 62,5% | 79,1% | < 10 ppm |

Für die Herstellung von Mischung 2 wird zwar kein aufwendig hergestellter Polymer-MDA-Typ A oder B und phosgeniertes Produkt (Polymer-Typ A und B) eingesetzt, dafür müssen aber 64 Gew.-% mit großem Aufwand hergestellte 4,4'-reine und 2,4'-reiche Typen eingesetzt werden. Die Herstellung von Mischung 2 nach Beispiel 2 ist daher energetisch sehr aufwendig. Sie besitzt aber gegenüber Mischung 1 einen deutlich niedrigeren 2,2'-MDI-Gehalt.

### Beispiel 3 (nicht erfindungsgemäß):

Herstellung eines MDI-Mischproduktes aus 35,9 % Polymer-Typ, 45,7 % Monomer Roh, 18,3 % 2,4-Typ ergibt die folgende Produktqualität

| Typ | 2,2'-MDI | 2,4'-MDI | 4,4'-MDI | Summe 2-Kern | MCB | PHI |
|---|---|---|---|---|---|---|
| Mischung 3 | 0,56% | 16,3% | 62,5% | 79,4% | 114 ppm | 22 ppm |

Das Beispiel 3 zeigt, dass mit Mischung 3 die wichtige Kundenanforderung an den Lösungsmittel-gehalt (< 20 ppm) und an den PHI-Gehalt (< 10 ppm) nicht erfüllt werden kann, die in der Mischung 2 (Beispiel 2) noch gegeben war. Der 2,2'-MDI-Gehalt kann jedoch ähnlich niedrig realisiert werden wie in Beispiel 2.

### Beispiel 4 (erfindungsgemäß):

Herstellung eines MDI-Mischproduktes aus 35,9 % Polymer-Typ, 45,7 % Monomer Roh rein, 18,3 % 2,4-Typ ergibt die folgende Produktqualität

| Typ | 2,2'-MDI | 2,4'-MDI | 4,4'-MDI | Summe 2-Kern | MCB | PHI |
|---|---|---|---|---|---|---|
| Mischung 4 | 0,56% | 16,3% | 62,5% | 79,4% | < 10 ppm | < 8ppm |

Nur ca. 18 % energieaufwändig feindestilliertes Isomerenprodukt wird für diese Mischungsherstellung benötigt, dies bedeutet eine Reduktion des Einsatzes an feindestillierten Isomerenprodukten um ca. 70 %. Auf die bei der MDA-Herstellung sehr aufwändigen Polymer-Typen A und B kann vollständig verzichtet werden. Bei unkritisch leicht erhöhtem 2,2'-Gehalt kann die vergleichbare Qualität wie im Beispiel 2 hergestellt werden.

### Beispiel 5 (erfindungsgemäß):

Falls tatsächlich der hohe 2,2'-Gehalt von 3,9 Gew.-% benötigt wird, lässt sich eine zusätzlich verfügbare Produktqualität (2,2'-reicher Typ) mit ca. 50 % 2,2'-MDI und ca. 50 % 2,4'-MDI als vierte Komponente einsetzen. Hiermit können die Ausgangswerte aus Beispiel 1 exakt eingestellt werden.

Herstellung eines MDI-Mischproduktes aus 30,2 % Polymer-Typ, 51,8 % Monomer Roh rein, 11,4 % 2,4-Typ und 6,7 % 2,2-Typ ergibt die folgende Produktqualität

| Typ | 2,2'-MDI | 2,4'-MDI | 4,4'-MDI | Summe 2-Kern | MCB | PHI |
|---|---|---|---|---|---|---|
| Mischung 5 | 3,9% | 16,3% | 62,5% | 82,7% | < 10 ppm | < 8 ppm |

Nur ca. 18 % feindestilliertes Produkt (2,2'-reicher Typ und 2,4'-reicher Typ) wird für die Herstellung von Mischung 5 benötigt. Auf die aufwändig hergestellten Polymer-Typen A und B kann vollständig verzichtet werden. Hierdurch ergibt sich eine Reduktion der energieaufwändig hergestellten Rohstoffe im Vergleich zu Beispiel 1 um fast 80 %.

### Beispiel 6 (nicht erfindungsgemäß):

Es werden 1000 g Anilin mit 306 g 31,9-%iger, wässriger HCl in einem Rührkessel bei 40°C vermischt. Hierzu wird in 15 Minuten 480 g 32 %ige Formaldehyd-Lösung zugetropft. Es wird zunächst weitere 15 min bei 40°C gerührt und die Temperatur langsam auf 100°C innerhalb der nächsten 2,5 h erhöht. Anschließend wird über 10 h bei 100°C am Rückfluss gerührt und die Reaktionsmischung mit 50 %iger Natronlauge neutralisiert, die wässrige Phase abgetrennt und die organische Phase mit Wasser nachgewaschen. Die organische Lösung wird entnommen und im Vakuum destillativ von überschüssigem Anilin befreit. Das MDA-Reaktionsprodukt wird in einem 2. Rührkessel in die vorgelegte eiskalte 15 %ige Lösung von Phosgen in Monochlorbenzol (MCB) gegossen; der molare Überschuss an Phosgen beträgt 150 %. Die Reaktionslösung wird unter kontinuierlicher Zudosierung von 40 Liter/h Phosgen langsam innerhalb einer Stunde auf 100°C aufgeheizt. Innerhalb einer weiteren Stunde wird das Gemisch auf Siedetemperatur gebracht, die Phosgendosierung gestoppt und Vakuum angelegt. Schrittweise wird die Temperatur auf 210°C erhöht, der Druck auf 3 mbar abgesenkt und das Lösungsmittel vollständig entfernt. Man erhält ein rohes MDI-Gemisch mit 58 Gew.-% monomerem MDI (davon 0,21 Gew.-% 2,2'-MDI, 5,1 Gew.-% 2,4'-MDI, 52,7 Gew.-% 4,4'-MDI), 65 ppm MCB und 14,5 ppm PHI.

Das rohe MDI-Gemisch wird in das polymere MDI-Produkt sowie die Monomer-Roh-Fraktion (2-Kern-MDI) zerlegt. Als Versuchsaufbau dient eine Glaskolonne mit einer Zuführung des Produktes auf den darunterliegenden Sumpfverdampfer, statt Trennböden enthält die Kolonne nur eine Tropfenabscheiderpackung. Am Kopf wird das Destillat vollständig niedergeschlagen und entnommen. Der Kopfdruck wird auf 5 mbar mittels einer Vakuumpumpe eingestellt.

Der Zulauf von 500 g/h rohem MDI-Gemisch wird mit 180°C der kontinuierlich betriebenen Apparatur zugeführt. Nach einer Einlaufphase von 2 h werden die folgenden Stoffströme als Produkte der Apparatur entnommen:
Sumpf der Destillationsapparatur: 62 g in 10 min mit der Zusammensetzung:
   0,09 % 2,2'-MDI, 3,7 % 2,4'-MDI, 39,8 % 4,4'-MDI, 0,5 ppm MCB, 4 ppm PHI, Rest: polymeres MDI, (Viskosität des Sumpfes bei 25°C: 185 mPas).
Kopfstrom der Destillationsapparatur: 21 g in 10 min mit der Zusammensetzung
   0,57 % 2,2'-MDI, 9,0 % 2,4'-MDI, 89,3 % 4,4'-MDI, 241 ppm MCB, 44 ppm PHI
   Alle %-Angaben der Zusammensetzungen beziehen sich auf das Gewicht der jeweiligen Gesamtprobe.

### Beispiel 7 (erfindungsgemäß):

Das rohe MDI-Gemisch aus Beispiel 6 wird in das polymere MDI-Produkt sowie das saubere Monomer-Roh rein und eine Leichtsiederfraktion zerlegt.

Als Versuchsaufbau dient eine Glaskolonne mit einer Zuführung des Produktes auf den Sumpfverdampfer, Edelstahl-Destillationspackung mit 4 theoretischen Trennstufen, wobei in der Mitte der Packung der flüssige Rückstrom vom Kopf der Laborkolonne entnommen und partiell wieder als Rücklauf auf die Kolonne zurückgeleitet werden kann. Am Kopf wird das Destillat vollständig niedergeschlagen und mittels eines Probenteilers zu 95 % wieder als Rücklauf auf die Kolonne gegeben. Der Kopfdruck wird auf 5 mbar mittels einer Vakuumpumpe eingestellt.

Der Zulauf von 500 g/h rohem MDI-Gemisch wird mit 180°C der kontinuierlich betriebenen Apparatur zugeführt. Nach einer Einlaufphase von 2 h werden die folgenden Stoffströme als Produkte der Apparatur entnommen:
Sumpf der Destillationsapparatur: 60 g in 10 min mit der Zusammensetzung
   0,08 % 2,2'-MDI, 3,6 % 2,4'-MDI, 39,9 % 4,4'-MDI, 0,5 ppm MCB, 4 ppm PHI, Rest: polymeres MDI, (Viskosität des Sumpfes bei 25°C: 205 mPas).
Kopfstrom der Destillationsapparatur: 2 g in 10 min mit der Zusammensetzung
   2,0 % 2,2'-MDI, 17,0 % 2,4'-MDI, 80,7 % 4,4'-MDI, 730 ppm MCB, 380 ppm PHI.

Seitenstrom der Destillationsapparatur zwischen den beiden Trennpackungselementen (entspricht der Fraktion Monomer Roh rein): 19 g in 10 min mit der Zusammensetzung 0,45 % 2,2'-MDI, 8,7 % 2,4'-MDI, 90,7 % 4,4'-MDI, 12 ppm MCB, 6 ppm PHI.

Alle %-Angaben der Zusammensetzungen beziehen sich auf das Gewicht der jeweiligen Gesamtprobe.

Obige Beispiele zeigen, dass die Herstellung von Abmischprodukten aus dem nach dem erfindungsgemäßen Verfahren hergestellten Monomer Roh rein entscheidende Vorteile gegenüber dem Stand der Technik bieten:
- die Gehalte an 2,2'-MDI im Abmischprodukt können durch Ersatz der Polymer-Typen A und B durch Monomer Roh rein bei der Abmischung deutlich reduziert werden, was zur Erhöhung der Reaktivität und einer Verringerung der Restmonomerengehalte führt.
- die Gehalte an Lösungsmittel wie MCB, Phenylisocyanat und Phosgen im Abmischprodukt können durch Ersatz von Monomer Roh durch Monomer Roh rein bei der Abmischung deutlich reduziert werden. Dadurch wird die Reinheit der Abmischprodukte und damit ihre Eigenschaften deutlich verbessert.

## Patentansprüche

1. Verfahren zur Herstellung einer Fraktion von Diisocyanaten der Diphenylmethanreihe enthaltend mindestens 95 Gew.-% 2-Kern-Methylendiphenyldiisocyanat, bei dem man
a) Anilin und Formaldehyd in Gegenwart eines sauren Katalysators zu Di- und Polyaminen der Diphenylmethanreihe enthaltend 2-Kern-Methylendiphenyldiamin umsetzt, und
b) die Di- und Polyamine der Diphenylmethanreihe enthaltend 2-Kern-Methylendiphenyldiamin gegebenenfalls in Gegenwart eines Lösungsmittels phosgeniert, wobei man ein rohes Di- und Polyisocyanat erhält, und
c) aus dem rohen Di- und Polyisocyanat eine Fraktion enthaltend mindestens 95 Gew.-% 2-Kern-Methylendiphenyldiisocyanat mit einem Gehalt an 4,4'-MDI größer 60 Gew.-%, einem Gehalt an 2,4'-MDI von 4 bis 35 Gew.-% und einem Gehalt an 2,2'-MDI von 0,01 bis 10 Gew.-%, bezogen auf die Masse der Fraktion, und maximal 20 ppm Phenylisocyanat sowie gegebenenfalls maximal 50 ppm Lösungsmittel abtrennt, wobei die Abtrennung der Fraktion in einem einzigen Destillationsschritt in einer Destillationskolonne mit Seitenstromentnahme, bei der die Fraktion im Seitenstrom der Kolonne und Leichtsieder am Kopf der Kolonne abgetrennt werden, erfolgt.

2. Verfahren gemäß Anspruch 1, bei dem man in Schritt c) aus dem rohen Di- und Polyisocyanat eine Fraktion enthaltend mindestens 99 Gew.-% 2-Kern-Methylendiphenyldiisocyanat mit einem Gehalt an 4,4'-MDI größer 76 Gew.-%, einem Gehalt an 2,4'-MDI von 5 bis 22 Gew.-% und einem Gehalt an 2,2'-MDI von 0,2 bis 3 Gew.-%, bezogen auf die Masse der Fraktion, und maximal 10 ppm, Phenylisocyanat sowie gegebenenfalls maximal 20 ppm Lösungsmittel abtrennt.

3. Verfahren zur Herstellung von Mischungen enthaltend Diisocyanate der Diphenylethanreihe, bei dem man
a) eine Fraktion enthaltend mindestens 95 Gew.-% 2-Kern-Methylendiphenyldiisocyanat mit einem Gehalt an 4,4'-MDI größer 60 Gew.-%, einem Gehalt an 2,4'-MDI von 4 bis 35 Ges.-% und einem Gehalt an 2,2'-MDI, von 0,01 bis 10 Gew.-%, bezogen auf die Masse der Fraktion, und maximal 20 ppm Phenylisocyanat sowie gegebenenfalls maximal 50 ppm Lösungsmittel nach dem Verfahren gemäß Anspruch 1 herstellt, und
b) die in Schritt a) hergestellte Fraktion mit einem oder mehreren Gemischen enthaltend aromatische Isocyanate abmischt.

4. Verfahren nach Anspruch 3, bei dem man
b) die in Schritt a) hergestellte Fraktion mit einem oder mehreren Gemischen enthaltend Di- und/oder Polyisocyanate der Diphenylmethanreihe abmischt.

5. Verfahren nach Anspruch 3, bei dem man
b) die in Schritt a) hergestellte Fraktion mit einem oder mehreren Gemischen enthaltend Toluylendiisocyanat abmischt.

6. Verfahren nach Anspruch 3, bei dem man
b) die in Schritt a) hergestellte Fraktion mit einem oder mehreren Gemischen enthaltend Naphthalindiisocyanat abmischt.

7. Verfahren zur Herstellung von Polymeren, bei dem man
a) eine Fraktion enthaltend mindestens 95 Gew.-% 2-Kern-Methylendiphenyldiisocyanat nach Anspruch 1 herstellt, oder
b) eine Mischung enthaltend Diisocyanate der Diphenylmethanreihe gemäß einem der Ansprüche 3 bis 6 herstellt und
c) die Fraktion aus Schritt a) und/oder die Mischung enthaltend Diisocyanate aus Schritt b) mit einer oder mehreren Polyolkomponenten zu einem Polymer umsetzt.

## Claims

1. A process for the production of a fraction of diisocyanates of the diphenylmethane series containing at least 95 wt.% binuclear methylenediphenyl diisocyanate comprising:
a) reacting aniline and formaldehyde in the presence of an acid catalyst to produce diamines and polyamines of the diphenylmethane series containing binuclear methylenediphenyl diamine,
b) phosgenating the diamines and polyamines of the diphenylmethane series containing binuclear methylenediphenyl diamine, optionally in the presence of a solvent, to produce a crude diisocyanate and polyisocyanate, and
c) separating from the crude diisocyanate and polyisocyanate a fraction containing at least 95 wt.% binuclear methylenediphenyl diisocyanate comprising greater than 60 wt.% 4,4'-MDI, from 4 to 35 wt.% 2,4'-MDI, and from 0.01 to 10 wt.% 2,2'-MDI relative to the mass of the fraction, and no more than 20 ppm phenyl isocyanate and optionally no more than 50 ppm of solvent, the separation of the fraction being effected in a single distillation step in a distillation column with sidestream withdrawal, in which the fraction is removed in the sidestream of the column and low boilers at the top of the column.

2. The process of Claim 1 in which a fraction containing at least 99 wt.% binuclear methylenediphenyl diisocyanate comprising greater than 76 wt.% 4,4'-MDI, from 5 to 22 wt.% 2,4'-MDI and from 0.2 to 3 wt.% 2,2'-MDI relative to the mass of the fraction, and a maximum of 10 ppm phenyl isocyanate and optionally a maximum of 20 ppm solvent is separated from the crude diisocyanate and polyisocyanate in step c).

3. A process for the production of mixtures containing diisocyantes of the diphenymethane series, comprising
a) producing a fraction comprising at least 95 wt.% binuclear methylenediphenyl diisocyanate comprising greater than 60 wt.% 4,4'-MDI, from 4 to 35 wt.% 2,4'-MDI, from 0.01 to 10 wt.% 2,2'-MDI relative to the mass of the fraction, and no more than 20 ppm phenyl isocyanate and optionally no more than 50 ppm solvent, by the process according to Claim 1, and
b) blending the fraction produced in step a) with one or more mixtures containing aromatic isocyanates.

4. The process of Claim 3 in which
b) the fraction produced in step a) is blended with one or more mixtures containing diisocyanates and/or polyisocyanates of the diphenylmethane series.

5. The process of Claim 3 in which
b) the fraction produced in step a) is blended with one or more mixtures containing toluene diisocyanate.

6. The process of Claim 3 in which
b) the fraction produced in step a) is blended with one or more mixtures containing naphthalene diisocyanate.

7. A process for the production of polymers comprising:
a) a fraction comprising at least 95 wt.% of binuclear methylenediphenyl diisocyanate according to Claim 1 is produced, or
b) a mixture comprising diisocyanates of the diphenylmethane series is prepared according to any of Claims 3 to 6 and
c) reacting the fraction from step a) and/or the blend containing diisocyanates from step b) with one or more polyol components to produce a polymer.

## Revendications

1. Procédé de fabrication d'une fraction de diisocyanates de la série du diphénylméthane contenant au moins 95 % en poids de diisocyanate de méthylène-diphényle binucléaire, selon lequel
a) de l'aniline et du formaldéhyde sont mis en réaction en présence d'un catalyseur acide pour former des di- et polyamines de la série du diphénylméthane contenant de la méthylène-diphényldiamine binucléaire, et
b) les di- et polyamines de la série du diphénylméthane contenant de la méthylène-diphényldiamine binucléaire sont éventuellement phosgénées en présence d'un solvant, un di- et polyisocyanate brut étant obtenu, et
c) une fraction contenant au moins 95 % en poids de diisocyanate de méthylène-diphényle binucléaire ayant une teneur en 4,4'-MDI supérieure à 60 % en poids, une teneur en 2,4'-MDI de 4 à 35 % en poids et une teneur en 2,2'-MDI de 0,01 à 10 % en poids, par rapport à la masse de la fraction, et au plus 20 ppm d'isocyanate de phényle, ainsi qu'éventuellement au plus 50 ppm de solvant est séparée du di- et polyisocyanate brut, la séparation de la fraction ayant lieu en une étape de distillation unique dans une colonne de distillation à soutirage de courant latéral, la fraction étant séparée dans le courant latéral de la colonne et les composants de point d'ébullition faible à la tête de la colonne.

2. Procédé selon la revendication 1, selon lequel une fraction contenant au moins 99 % en poids de diisocyanate de méthylène-diphényle binucléaire ayant une teneur en 4,4'-MDI supérieure à 76 % en poids, une teneur en 2,4'-MDI de 5 à 22 % en poids et une teneur en 2,2'-MDI de 0,2 à 3 % en poids, par rapport à la masse de la fraction, et au plus 10 ppm d'isocyanate de phényle, ainsi qu'éventuellement au plus 20 ppm de solvant, est séparée à l'étape c) du di- et polyisocyanate brut.

3. Procédé de fabrication de mélanges contenant des diisocyanates de la série du diphénylméthane, selon lequel
a) une fraction contenant au moins 95 % en poids de diisocyanate de méthylène-diphényle binucléaire ayant une teneur en 4,4'-MDI supérieure à 60 % en poids, une teneur en 2,4'-MDI de 4 à 35 % en poids et une teneur en 2,2'-MDI de 0,01 à 10 % en poids, par rapport à la masse de la fraction, et au plus 20 ppm d'isocyanate de phényle, ainsi qu'éventuellement au plus 50 ppm de solvant, est fabriquée par le procédé selon la revendication 1, et
b) la fraction fabriquée à l'étape a) est mélangée avec un ou plusieurs mélanges contenant des isocyanates aromatiques.

4. Procédé selon la revendication 3, selon lequel
b) la fraction fabriquée à l'étape a) est mélangée avec un ou plusieurs mélanges contenant des di- et/ou polyisocyanates de la série du diphénylméthane.

5. Procédé selon la revendication 3, selon lequel
b) la fraction fabriquée à l'étape a) est mélangée avec un ou plusieurs mélanges contenant du diisocyanate de toluylène.

6. Procédé selon la revendication 3, selon lequel
b) la fraction fabriquée à l'étape a) est mélangée avec un ou plusieurs mélanges contenant du diisocyanate de naphtaline.

7. Procédé de fabrication de polymères, selon lequel
a) une fraction contenant au moins 95 % en poids de diisocyanate de méthylène-diphényle binucléaire est fabriquée selon la revendication 1, ou
b) un mélange contenant des diisocyanates de la série du diphénylméthane est fabriqué selon l'une quelconque des revendications 3 à 6, et
c) la fraction de l'étape a) et/ou le mélange contenant des diisocyanates de l'étape b) sont mis en réaction avec un ou plusieurs composants polyol pour former un polymère.
